# NEUE EUROPÄISCHE PATENTSCHRIFT

(11) **EP 2 190 402 B2**
(45) Veröffentlichungstag und Bekanntmachung des Hinweises auf die Entscheidung über den Einspruch: **17.10.2018**
(45) Hinweis auf die Patenterteilung: 13.01.2016
(21) Anmeldenummer: 08774106.2
(22) Anmeldetag: 17.06.2008
(51) Int. Cl.: A61K 8/25, A61K 8/67, A61Q 19/10, A61Q 5/02, A61Q 5/04, A61Q 5/06, A61Q 5/10, A61Q 5/12, A61K 8/44, A61Q 5/00, A61K 8/64

(54) **BIOTIN UND KIESELSÄURE IN FÄRBEMITTELN**
BIOTIN AND SILICA IN DYE COMPOSITIONS
BIOTINE ET SILICE DANS DES COMPOSITIONS COLORANTES

(30) Priorität: 25.09.2007 DE 102007045974
(43) Veröffentlichungstag der Anmeldung: 02.06.2010
(73) Patentinhaber: Henkel AG & Co. KGaA, 40589 Düsseldorf (DE)
(72) Erfinder: KLEEN, Astrid, 22457 Hamburg (DE); TERRIER, Janie, 22589 Hamburg (DE); AKRAM, Mustafa, 22455 Hamburg (DE)
(86) Internationale Anmeldenummer: PCT/EP2008/057596
(87) Internationale Veröffentlichungsnummer: WO 2009/040149

(56) Entgegenhaltungen:
- EP-A- 1 541 152
- WO-A-97/02041
- WO-A-98/06376
- WO-A-2005/020945
- WO-A-2006/032374
- WO-A-2006/066323
- WO-A-2007/003307
- DE-A1- 19 903 241
- DE-A1- 19 962 869
- DE-A1-102004 045 274
- DE-U1- 20 220 180
- US-A1- 2002 028 257
- US-A1- 2002 086 039

## Beschreibung

Die vorliegende Anmeldung betrifft Mittel für keratinische Fasern, enthaltend eine Kombination aus mindestens einer Biotinverbindung in einer Menge von 0,0001 bis 0,01 Gew.-% und mindestens einer gegebenenfalls hydratisierten SiO₂-Verbindung. Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Haarpflege und gegebenenfalls gleichzeitigen Veränderung oder Nuancierung der Farbe des Kopfhaares eine wichtige Rolle.

Um den Pflegezustand der Fasern zu verbessern, ist es seit langem üblich, die Fasern, insbesondere im Anschluss an die farbverändernde Behandlung, einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splissrate verringert. Es wird zwischen solchen Präparaten unterschieden, die überwiegend auf der Haaroberfläche (der Cuticula) aktiv sind, solchen, die in der Haarfaser (dem Cortex) ihre Wirkung entfalten und solchen, die Wirkstoffe in die Haarwurzel bringen und so das Haarwachstum beeinflussen.

In jüngster Zeit wurden ferner sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den Komponenten, beispielsweise zur Färbung oder zur permanenten Umformung der Haare, zusätzlich Wirkstoffe, die den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die im Rahmen derartiger Kombinationspräparate einsetzbaren Wirkstoffe müssen insbesondere hinsichtlich ihrer Stabilität hohen Ansprüchen genügen, da beispielsweise die Färbecremes meist einen hohen pH-Wert und die Oxidationsmittelzubereitungen einen niedrigen pH-Wert aufweisen. Ferner sind Unverträglichkeiten der verschiedenen Wirkstoffe untereinander und somit eine geringe Lagerstabilität zu vermeiden.

Im Rahmen der Anmeldungen DE-A-199 14 927, DE-A-199 14 926 und DE-A-44 08 506 wurden bereits derartige Wirkstoffkombinationen zum Einsatz in oxidativen Färbemitteln vorgeschlagen. Dennoch lassen auch diese Mittel, insbesondere auf schwer zu pflegenden Fasern, wie beispielsweise japanischem Haar, noch einige Wünsche hinsichtlich der pflegenden Eigenschaften offen.

WO 2005/020945 A1 offenbart Mittel zur Aufhellung keratinhaltiger Fasern, die einen pH-Wert von 4.5 bis 9 aufweisen und mindestens eine Peroxoverbindung sowie Wasserstoffperoxid und mindestens eine gegebenenfalls hydratisierte SiO2-Verbindung enthalten. Optional ist Biotin als Struktur verbessernder Wirkstoff enthalten.

EP 1541152 A1 beschäftigt sich mit Hautkosmetika zum Schutz und zur Pflege der Haut, insbesondere empfindlicher Haut. Beispiel 8 enthält Biotin und eine SiO2-Verbindung, die als Füllstoff bzw. als Additiv wirkt.

WO 98/06376 A1 beschäftigt sich mit kosmetischen Mitteln zur Stärkung der Nägel. Biotin ist als Vernetzer für die Schwefel-Schwefel-Bindung des Nagelproteins offenbart.

US 2002/0086039 A1 offenbart wasserfreie kosmetische Zusammensetzungen, die bioaktives Glas enthalten, wobei das bioaktive Glas antibakteriell, antifungal, antiviral, Ionen-freisetzend, entzündungshemmend oder pH-regulierend wirkt.

DE 19903241 A1 beschäftigt sich mit Hautkosmetika zum Schutz und zur Pflege der Haut, die Biotin und Harnstoff enthalten.

US 2002/0028257 A1 und WO 2006/066323 A1 offenbaren Zusammensetzungen, die unter anderem Biotin und Silica als Haarwuchs fördernde Wirkstoffe enthalten.

DE 20220180 U1 offenbart Nahrungsergänzungsmittel, die unter anderem Biotin und Kieselsäure als Wirkstoffe enthalten.

WO 97/02041 A1 offenbart transdermale und orale Zusammensetzungen zur Behandlung der androgenen Alopezie, die diverse Antiandrogene enthält.

Die Komponenten, die als farbgebende Komponenten üblicherweise in Haarfärbemitteln genutzt werden, sind nachfolgend genannte:

Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Es ist ein allgemeines Bestreben, die mit den bekannten Methoden erzielbaren Färbungen in Ihrem Farbergebnis zu intensivieren. Auf diese Weise kann entweder mit der gleichen Farbstoffmenge eine intensivere Färbung erzielt werden, oder es lässt sich unter Einsparung von Farbstoffen ein gewünschter Intensitätsgrad einer Färbung beibehalten. Letzteres trägt auch zur Wirtschaftlichkeit von Färbemitteln bei. Desweiteren sollen erzielte Färbungen ein hohes Maß an Farbechtheit z.B. gegen Waschen, Licht oder Reibung aufweisen und müssen insbesondere im Rahmen der Haarpflege kompatibel mit anderen Haarbehandlungsmitteln sein. Besonders sollen, insbesondere die oxidativen Farbveränderungsmittel, die Haarstruktur schonen.

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung der keratinreduzierenden Substanz ist üblicherweise alkalisch eingestellt, damit zum einen ein genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu - SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist oftmals ein Verspröden und Stumpfwerden der Haare. Diese Begleiterscheinung bedeutet schlimmstenfalls eine beschleunigte Haaralterung, insbesondere eine dauerhafte Schädigung der Haarfaser, die zu Haarbruch oder Haarspliß führen kann.

Es hat in der Vergangenheit nicht an Versuchen gefehlt, Wellmittel zu formulieren, welche die Struktur der Haarfaser schonen, die Haaralterung verringern und ein hervorragendes Wellergebnis liefern.

Es bestand ebenso die Aufgabe, ein Wellmittel für die dauerhafte Verformung von keratinhaltigen Fasern bereitzustellen, nach dessen Anwendung die genannten unerwünschten Nebenwirkungen der keratinreduzierenden Substanzen verringert oder ganz ausgeschlossen werden.

Zusammenfassend besteht weiterhin ein Bedarf an pflegenden Wirkstoffen, insbesondere für die Kombination mit einer farbverändernden bzw. dauerhaft umformenden Behandlung von keratinhaltigen Fasern. Somit war es die Aufgabe der vorliegenden Erfindung, ein Mittel zu entwickeln, dass die Haaralterung mindert und, wenn es im Rahmen eines Kombinationspräparates mit einer Farbveränderung oder Dauerverformung angewendet wird, die Farbgebung bzw. Umformungswirkung intensiviert und die Haltbarkeit der Färbung bzw. der Umformung gegen äußere Einflüsse verbessert.

Es wurde nunmehr überraschenderweise gefunden, dass Mittel, die mindestens eine Biotinverbindung in einer Menge von 0,0001 bis 0,01 Gew.-% und mindestens eine gegebenenfalls hydratisierte SiO₂-Verbindung sowie zusätzlich mindestens ein Oxidationsfarbstoffvorprodukt und/oder mindestens einen direktziehenden Farbstoff enthalten, eine ausgezeichnete Pflegewirkung entfalten und der Haaralterung vorbeugen. Im Rahmen dieser Ausführungsform lassen sich hervorragende farbechte Färbungen mit exzellenter Intensität erzielen. Ausserdem wird im Rahmen der Ausführungsform als Wellmittel zusätzlich ein hervorragendes Wellergebnis erhalten.

Unter Haaralterung wird erfindungsgemäß sowohl die Alterung der Faserstruktur und der Oberfläche des Haars als auch die vorzeitige Einleitung der Apoptose, das heißt des programmierten Zelltods im Haarfollikel, subsumiert.

Ein erster Gegenstand der vorliegenden Anmeldung ist daher ein Mittel zur Behandlung keratinischer Fasern, insbesondere menschlicher Haare, das in einem kosmetischen Träger
(A) mindestens eine Biotinverbindung der Formel (I) 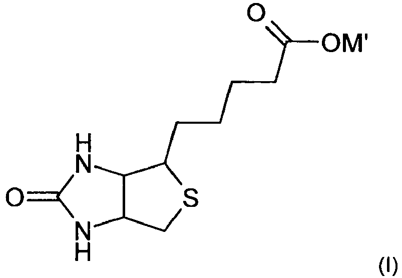 worin M' für ein ein Wasserstoffatom, eine (C₁ bis C₆)-Alkylgruppe, eine (C₂ bis C₆)-Hydroxyalkylgruppe, eine (C₂ bis C₆)-Alkenylgruppe, ein Äquivalent eines ein- oder mehrwertigen Kations steht,
   wobei die Biotinverbindung in einer Menge von 0,0001 Gew.-% bis 0,01 Gew.-%, bezogen auf das Gewicht des Mittels, enthalten ist
   und
(B) mindestens eine gegebenenfalls hydratisierte SiO₂-Verbindung enthält,
   wobei zusätzlich mindestens ein Oxidationsfarbstoffvorprodukt und/oder mindestens ein direktziehender Farbstoff enthalten ist.

Unter "keratinischen Fasern" sind dabei erfindungsgemäß Pelze, Wolle, Federn und insbesondere menschliche Haare zu verstehen.

Die Mittel umfassen einen kosmetischen Träger. Solche kosmetische Träger sind beispielsweise Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in einen pulverförmigen oder auch tablettenförmigen kosmetischen Träger zu integrieren, welcher vor der Anwendung in Wasser gelöst wird. Die kosmetischen Träger können insbesondere wässrig oder wässrig-alkoholisch sein.

Ein wässriger Träger enthält mindestens 50 Gew.-% Wasser.

Unter wässrig-alkoholischen Trägern sind im Sinne der vorliegenden Erfindung wässrige Lösungen enthaltend 3 bis 70 Gew.-% eines C₁-C₄-Monohydroxyalkohols, insbesondere Ethanol bzw. Isopropanol, zu verstehen. Die erfindungsgemäßen Mittel können zusätzlich weitere organische Lösemittel, insbesondere C₁-C₄-Polyhydroxyalkohole wie beispielsweise Ethyldiglykol, Glyzerin oder 1,2-Propylenglykol, enthalten. Bevorzugt sind dabei alle wasserlöslichen organischen Lösemittel.

Im Rahmen der Gruppe M' der Biotinverbindungen gemäß Formel (I) stehen die (C₁ bis C₆)-Alkylgruppen bevorzugt für -CH₃, -CH₂CH₃, -CH(CH₃)₂, -CH₂CH₂CH₃, -CH(CH₃)CH₂CH₃, -CH₂CH(CH₃)₂, -C(CH₃)₃,-CH₂CH₂CH₂CH₃, -CH₂CH₂CH₂CH₂CH₃, -CH₂CH₂CH₂CH₂CH₂CH₃.

Im Rahmen der Gruppe M' der Biotinverbindungen gemäß Formel (I) stehen die (C₂ bis C₆)-Hydroxyalkylgruppen bevorzugt für -CH₂CH₂OH, -CHOHCH₃, -CH₂CH₂CH₂OH, -CH₂CHOHCH₃.

Im Rahmen der Gruppe M' der Biotinverbindungen gemäß Formel (I) stehen die (C₂ bis C₆)-Alkenylgruppen bevorzugt für -CH=CH₂, -CH₂CH=CH₂, -CH₂CH₂CH=CH₂.

Wenn die Verbindungen der Formel (I) als Säure vorliegen, bedeutet der Rest M' ein Wasserstoffatom. Wenn die Verbindungen der Formel (I) als Salz vorliegen, steht M' für ein Äquivalent eines ein- oder mehrwertigen Kations.

Das ein oder mehrwertige Kation M'<y+>mit einer Ladungszahl y von eins oder höher dient lediglich aus Gründen der Elektroneutralität zur Kompensation der einfach negativen Ladung des bei Salzbildung vorliegenden Carboxylatfragments -COO<(-)>der Formel (I). Das dafür zu verwendende Äquivalent des entsprechenden Kations beträgt 1/y. Das Fragment -COOM' der Formel (I) steht im Fall der Salzbildung für die Gruppe:

-COO(⁻)1/y(M'y⁺)

Als ein- oder mehrwertige Kationen M' y⁺ kommen prinzipiell alle physiologisch verträglichen Kationen in Frage. Insbesondere sind dies Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol.

M' steht in der Formel (I) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Als bevorzugte Biotinverbindung gemäß Formel (I) gilt Biotin und/oder mindestens eines seiner Salze. Unter Biotin als solches versteht man (3aS,4S,6aR)-2-Oxohexahydrothienol[3,4-d]-imidazol-4-valeriansäure (in Formel (I) steht M' = H). Die Verbindung wird auch als Vitamin H bzw. Vitamin B₇ bezeichnet.

Die Biotinverbindungen der Formel (I) sind in dem erfindungsgemäßen Mittel bevorzugt in einer Menge von 0,0005 Gew.-% bis 0,01 Gew.-%, bezogen auf das anwendungsbereite Mittel, enthalten.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von (i) polymeren Kieselsäuren, d.h. in Form von Kieselsäuregel (Silicagel) oder Kieselsol, oder (ii) Wasserglas
eingesetzt.

Kieselsäuregele werden erfindungsgemäß aus kolloid-dispersen Kiselsäureteilchen gebildet. Kielelsole werden aus kolloid gelösten Kieselsäureteilchen mit einem Teilchendurhcmesser von 5 bis 150 nm gebildet. Als Kieselsäureteilchen eignen sich besonders bevorzugt solche Kieselsäure n, die pyrogen hergestellt wurden. Diese Kieselsäuren werden durch Flammenhydrolyse von SiCl4in einer Knallgasflamme beispielsweise bei 1000°C synthetisiert:

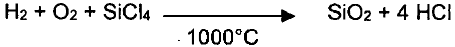

Bevorzugt geeignete Kieselsäuren besitzen bevorzugt eine BET-Oberfläche von 50 bis 400 m²/g (gemessen nach DIN 66131 mit Stickstoff).

Wiederum sind solche Kieselsäuren bevorzugt, deren mittlerer Teilchendurchmesser in einem Bereich von 10 nm bis 50 µm, insbesondere in einem Bereich von 1 µm bis 20 µm liegt.

Bevorzugt geeignete Kieselsäuren werden unter dem Handelsnamen Aerosil® von der Fa. Degussa vertrieben.

Erfindungsgemäß besonders bevorzugt sind Wassergläser, die aus einer wäßrigen Lösung eines Silicates der Formel (SiO₂)ₙ(Na₂O)ₘ(K₂O)ₚgebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten. Erfindungsgemäß besonders bevorzugte Wassergläser werden unter anderem von der Fa. Henkel unter den Bezeichnungen Ferrosil® 119, Natronwasserglas 40/42, Portil ®A, Portil® AW und Portil® W und von der Firma Akzo unter der Bezeichnung Britesil® C20 vertrieben.

Obwohl bereits geringe Mengen der gegebenenfalls hydratisierten SiO₂-Verbindungen die Farbintensivierung bewirken, kann es erfindungsgemäß bevorzugt sein, die gegebenenfalls hydratisierten SiO₂-Verbindungen in Mengen von 0,005 Gew.-% bis 5 Gew.-%, besonders bevorzugt in Mengen von 0,05 Gew.-% bis 2 Gew.-%, weiter bevorzugt in Mengen von 0,1 Gew.-% bis 1,0 Gew.-% und ganz besonders bevorzugt in Mengen von 0,2 Gew.-% bis 1,0 Gew.-%, jeweils bezogen auf das Mittel, einzusetzen. Die Mengenangaben geben dabei jeweils den Gehalt der SiO₂-Verbindungen (ohne Wasseranteil) in den Anwendungszubereitungen wieder.

Besonders vorteilhaft haben sich erfindungsgemäße Mittel erwiesen, die die Biotinverbindungen der Formel (I) in Relation zu den gegebenenfalls hydratisierten SiO₂-Verbindungen in einem Gewichtsverhältnisbereich von 500 zu 1 bis 1 zu 1, insbesondere von 100 zu 1 bis 20 zu 1, enthalten.

In einer erfindungsgemäß bevorzugten Ausführungsform enthält das erfindungsgemäße Mittel zusätzlich mindestens ein Protein des Samens der Gattung der Moringagewächse.

Unter dem Begriff "Protein des Samens der Gattung der Moringagewächse" sind erfindungsgemäß neben den Proteinen in ihrer natürlichen Form auch die durch chemische und/oder enzymatische Umwandlung herstellbaren Derivate dieser Proteine, wie beispielsweise die Proteinhydrolysate und die quaternisierten Derivate des Samens dieser Pflanzengattung, umfasst.

Es ist bereits seit langem bekannt, in kosmetischen Präparaten Proteine oder modifizierte Proteine zur Erzielung von pflegenden Effekten einzusetzen. Zu diesem Zweck werden entweder wasserlösliche Proteine oder durch chemische und/oder durch enzymatische Reaktionen modifizierte, also wasserlöslich gemachte Proteine eingesetzt. Gerade bei den Umsetzungen zur Erzielung einer ausreichenden Wasserlöslichkeit ist bei Faserproteinen häufig ein so weitgehender Abbau erforderlich, dass die kosmetische Wirksamkeit nicht mehr ausreichend ist. In jüngster Zeit werden immer häufiger Pflanzenproteine und deren Hydrolysate sowie Derivate in der Kosmetik verwendet. Bekannt sind beispielsweise Produkte auf der Basis von Weizen, Hafer, Reis, Mais, Kartoffeln oder Soja.

Zu den Pflanzen, welche interessante wirksame Inhaltsstoffe enthalten, gehört auch die Familie der Moringagewächse. Moringagewächse sind bereits seit dem Altertum bekannt. Besser bekannt sind Gewächse dieser Art unter ihrem Trivialnamen "Wunderbaum". Sie sind bevorzugt in tropischen Gebieten beheimatet. Die verschiedenen Teile dieser Pflanzengattung werden bereits seit dem Altertum insbesondere zu medizinischen Zwecken verwendet.

Zur Gattung der Moringagewächse zählen etwa 14 Arten. Eine davon ist Moringa oleifera (Moringa pterygosperma). Weitere Arten sind beispielsweise Moringa drouhardii, Moringa concanensis oder Moringa peregrina. Aus den Samen der Moringagewächse wird durch eine schonende Extraktion mit Wasser und Glycerin das Protein gewonnen. Dieses Protein hat ein Molgewicht von 500 bis 50.000 Dalton. Bevorzugt ist ein Proteinextrakt mit einem Molgewicht von 3000 bis 30.000 Dalton, ganz besonders bevorzugt von 5000 bis 15.000 Dalton. Erfindungsgemäß besonders bevorzugt ist ein Extrakt, der aus der Pflanze Moringa Oleifera gewonnen wird. Weiterhin kann der erfindungsgemäße Extrakt aufgrund der Extraktion selbstverständlich Wasser und Glycerin enthalten. Der Gehalt an extrahiertem Protein im Extrakt beträgt 0,01 bis 20 Gew.%. Ein Gehalt an Protein von 0,01 bis zu 10 Gew.% ist dabei bevorzugt. Besonders bevorzugt ist ein Extrakt mit einem Proteingehalt von 0,01 bis zu 5 Gew.%. Weiterhin sind in dem Extrakt mindestens 30 Gew.% Glycerin enthalten. Schließlich ist Wasser in dem erfindungsgemäßen Extrakt enthalten. Ein derartiges Protein ist beispielsweise unter der Handelsbezeichnung Puricare® LS 9658 von der Fa. Laboratoires Sérobiologiques im Handel erhältlich.

In den kosmetischen Zusammensetzungen ist der zuvor beschriebene Proteinextrakt aus den Samen der Moringagewächse bevorzugt in einer Menge von mindestens 0,01 bis zu 20 Gew.% enthalten. Bevorzugt werden Mengen des Extraktes von 0,01 bis zu 10 Gew.%, ganz besonders bevorzugt Mengen von 0,01 bis 5 Gew.% bezogen auf die gesamte kosmetische Zusammensetzung verwendet.

In einer bevorzugten Ausführungsform ließ sich die erfindungsgemäße Wirkung steigern, in dem das erfindungsgemäße Mittel zusätzlich als weitere Komponente mindestens eine Verbindung der Formel (II) enthält, 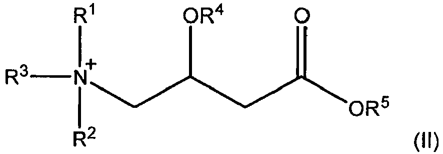 worin
- R¹, R² und R³: unabhängig voneinander für eine C₁- bis C₄-Alkylgruppe oder eine C₂- bis C₄-Hydroxyalkylgruppe stehen,
- R⁴: ein Wasserstoffatom, eine C₁- bis C₄-Alkylgruppe oder eine C₂- bis C₆-Acylgruppe bedeutet,
- R⁵: für ein Wasserstoffatom, eine negative Ladung oder eine C₁- bis C₄-Alkylgruppe steht.

Die Auswirkung von Stressfaktoren (wie die Auswirkung des oxidativen Stresses, (zum Beispiel verursacht durch freie Radikale), Stress durch UV-Strahlung, Stress durch Alterungsprozesse, psychischer Stress oder Stress durch nicht physiologische pH-Werte) auf den Stoffwechsel des Haars bzw. der Haarfollikelzellen, wird durch den Zusatz der Verbindung gemäß Formel (II) zusätzlich verhindert oder vermindert. Dabei wird die durch Stressfaktoren begünstigte bzw. beschleunigte Apoptose der Haarfollikelzellen verhindert oder vermindert. Die durch Stressfaktoren begünstigte Haaralterung, das heißt das vorzeitige Absterben des Haars bzw. der vorzeitige Ausfall des Haars wird auf diese Weise verhindert oder vermindert.

Stressfaktoren sind im Sinne der Erfindungen solche inneren oder äußeren Faktoren bzw. Reize, die eine vorzeitige Apoptose einleiten.

Die Verbindungen der Formel (II) besitzen bevorzugt ein Molekulargewicht von weniger als 500 g/mol, besonders bevorzugt von weniger als 400 g/mol.

In Formel (II) stehen die Reste R¹, R² und R³ bevorzugt unabhängig voneinander für eine Methyl- oder eine Ethylgruppe, besonders bevorzugt für eine Methylgruppe.

Der Rest R⁴ der Formel (II) steht bevorzugt für ein Wasserstoffatom oder eine C₂- bis C₄-Acylgruppe. Eine bevorzugte C₂- bis C₄-Acylgruppe ist die Acetylgruppe.

Wenn der Rest R⁵ für eine negative Ladung steht, liegen die Verbindungen der Formel (II) als inneres Salz vor. Wenn R<5>für ein Wasserstoffatom oder eine C₁- bis C₄-Alkylgruppe steht, liegt die Verbindung der Formel (II) als Salz mit einem physiologisch verträglichen Anion X- als Gegenion vor. Aktives Prinzip gemäß dieser Ausführungsform der Erfindung ist die Struktureinheit mit der Formel (II).

Die physiologisch verträglichen Anionen X- dürfen definitionsgemäß nicht nur eine negative Ladung tragen, sondern können auch eine Ladungszahl größer 1 besitzen. In letzterem Falle werden die Anionen

X- der Salzform zur Wahrung der Elektroneutralität durch Formulierung eines stöchiometrischen Koeffizienten kleiner 1 vor der Bezeichnung des Anions beschrieben. Die physiologisch verträglichen Anionen werden bevorzugt ausgewählt aus Halogenid, 0.5 Sulfat, Hydrogensulfat, 0.5 Carbonat, Hydrogencarbonat, 1/3 Phosphat, 0.5 Hydrogenphosphat, Dihydrogenphosphat, Carboxylat, wie beispielsweise Lactat, Citrat oder Tartrat. Besonders bevorzugt steht X⁻ für Chlorid, Bromid oder ein Carboxylat-Gegenion, insbesondere für Lactat, Citrat oder Tartrat.

Das beta-Kohlenstoffatom des Buttersäuregrundgerüsts der Verbindungen der Formel (II) ist chiral. Der Gegenstand der Erfindung betrifft sowohl Verbindungen der Formel (II) in L-Konfiguration, als auch Verbindungen der Formel (II) in D-Konfiguration sowie Gemische beider Konfigurationen, wie beispielsweise Racemate.

Beispiele für eine (C₂- bis C₄)-Hydroxyalkylgruppe der Formel (II) sind 2-Hydroxyethyl, 2-Hydroxypropyl, 3-Hydroxypropyl und 4-Hydroxybutyl.

Beispiele für eine (C₁- bis C₄)-Alkylgruppe der Formel (II) sind Methyl, Ethyl, n-Propyl, iso-Propyl, n-Butyl, iso-Butyl, sec-Butyl und tert-Butyl.

Beispiele für eine (C₂- bis C₆)-Acylgruppe der Formel (II) sind Acetyl, Propionyl, Butyryl und Valeryl. Bevorzugt handelt es sich bei der erfindungsgemäß verwendeten Verbindung gemäß Formel (II) um mindestens eine Verbindung, ausgewählt aus der Gruppe L-Carnitin, D-Carnitin, D,L-Carnitin, L-Carnitintartrat, D-Carnitintartrat, D,L-Carnitintartrat, L-Carnitinlactat, D-Carnitinlactat, D,L-Carnitinlactat, L-Carnitincitrat, D-Carnitincitrat, D,L-Carnitincitrat, L-Acetylcarnitin, D-Acetylcarnitin und D,L-Acetylcarnitin. Die Verbindungen der Formel (II) sind in dem erfindungsgemäßen Mittel dieser Ausführungsform bevorzugt in einer Menge von 0,001 bis 10 Gew.-%, besonders bevorzugt von 0,01 bis 5 Gew.-%, ganz besonders bevorzugt von 0,1 bis 2 Gew.-%, jeweils bezogen auf das Gewicht des Mittels, in den erfindungsgemäßen Mitteln enthalten.

Ein erfindungsgemäßes Mittel enthält folglich bevorzugt in einem kosmetischen Träger
(A) mindestens eine Biotinverbindung der Formel (I) 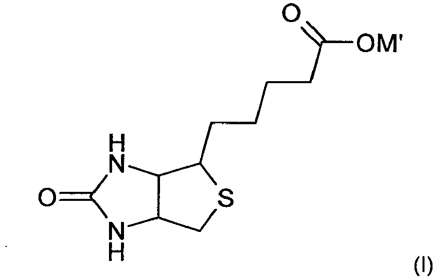 worin M' für ein Wasserstoffatom, eine (C₁bis C₂₀)-Alkylgruppe, eine (C₂bis C₂₀)-Hydroxyalkylgruppe oder ein Äquivalent eines ein- oder mehrwertigen Kations steht,
   wobei die Biotinverbindung in einer Menge von 0,0001 Gew.-% bis 0,01 Gew.-%, bezogen auf das Gewicht des Mittels, enthalten ist
(B) mindestens eine gegebenenfalls hydratisierte SiO2-Verbindung und zusätzlich mindestens ein Oxidationsfarbstoffvorprodukt und/oder mindestens einen direktziehenden Farbstoff und
(C)
   (i) mindestens ein Protein des Samens der Gattung der Moringagewächse, insbesondere aus dem Samen der Moringa oleifera,
      und/oder
   (ii) mindestens eine Verbindung der Formel (II) 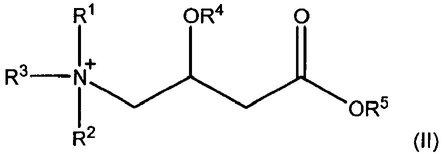 worin
      - R¹, R² und R³: unabhängig voneinander für eine C₁- bis C₄-Alkylgruppe oder eine C₂- bis C₄-Hydroxyalkylgruppe stehen,
      - R⁴: ein Wasserstoffatom, eine C₁- bis C₄-Alkylgruppe oder eine C₂- bis C₆-Acylgruppe bedeutet,
      - R⁵: für ein Wasserstoffatom, eine negative Ladung oder eine C₁- bis C₄-Alkylgruppe steht.

Im Rahmen einer bevorzugten Ausführungsform eines ein Kombinationspräparats zur Haarpflege und gleichzeitiger dauerhafter Haarumformung ist das erfindungsgemäße Mittel als Wellmittel konfektioniert und enthält neben der erfindungsgemäßen Wirkstoffkombination bzw. deren bevorzugten Ausführungsformen, zusätzlich mindestens eine keratinreduzierende Verbindung.

Die zusätzlichen keratinreduzierenden Verbindungen werden bevorzugt ausgewählt aus mindestens einem Vertreter der Gruppe, die gebildet wird aus
- Mercaptanen mit mindestens einer Gruppe -SH und
- Sulfit-Verbindungen.

Bevorzugte Sulfitverbindungen werden ausgewählt aus mindesetns einem Vertreter der Gruppe, die gebildet wird aus Sulfiten, Hydrogensulfiten oder Disulfiten. Beispiele für keratinreduzierende Verbindungen der Disulfite sind Natriumdisulfit (Na₂S₂O₅), Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite sind Sulfite als Alkali-, Ammonium- oder Alkanolammonium-Salz auf Basis eines C₂-C₄-Mono-, Di- oder Trialkanolamins. Ammoniumsulfit ist dabei bevorzugt.

Bevorzugte keratinreduzierende Verbindungen der Gruppe der Mercaptane sind Thiosäuren, das heißt Verbindungen, die (i) mindestens eine Gruppe -SH und (ii) mindestens eine Sulfonsäure oder Sulfonatgruppe oder Carboxylgruppe oder Carboxylatgruppe tragen. Dabei sind wiederum solche Thiosäuren bevorzugt, die (i) mindestens eine Gruppe -SH, (ii) mindestens eine mindestens eine Carboxylgruppe oder Carboxylatgruppe und (iii) mindestens eine Hydroxygruppe und/oder Aminogruppe tragen. Besonders bevorzugte Vertreter der Thiosäuren sind Thioglykolsäure, Thiomilchsäure, Thioäpfelsäure, Cystein, Mercaptoethansulfonsäure sowie die Salze und Ester der vorgenannten Thiosäuren. Der Einsatz mindestens einer Thiosäure als zusätzliche keratinreduzierende Verbindung ist erfindungsgemäß bevorzugt.

Die zusätzlichen keratinreduzierenden Verbindungen werden in den erfindungsgemäßen Wellmitteln bevorzugt in Konzentrationen von 0,2 bis 15 Gew.-%, bezogen auf das Gewicht des Wellmittels, eingesetzt. Der pH-Wert des erfindungsgemäßen Wellmittels liegt im Bereich von pH 5 bis 12, insbesondere von pH 7 bis 9,5. Der pH-Wert der erfindungsgemäßen Wellmittel wird bei Verwendung von Sulfit und/oder Disulfit und/oder Hydrogensulft bevorzugt auf einen Wert im Neutralbereich von pH 5 bis 8, bevorzugt von pH 6 bis 7.5 eingestellt.

Eine erfindungsgemäße Ausführungsform der Erfindung ist ein Kombinationspräparat zur Haarpflege und gleichzeitigen Farbveränderung keratinhaltiger Fasern, insbesondere menschlicher Haare. Es wurde überraschenderweise gefunden, dass sich die Wirkstoffkombination in Gegenwart von zusätzlich mindestens einem Oxidationsfarbstoffvorprodukt und/oder mindestens einem direktziehenden Farbstoff positiv auf die Intensität der Farbveränderung auf keratinhaltigen Fasern, insbesondere aus grauem Humanhaar, auswirkt. Es werden gleichmäßige, beständige und ausdruckstarke Färbungen der keratinhaltigen Fasern, insbesondere des grauen Haars, erhalten. Diese Färbungen erweisen sich als farbecht. Unter Oxidationsfarbstoffvorprodukt im Sinne der vorliegenden Erfindung wird ein Oxidationsfarbstoffvorprodukt vom Typ der Entwicklerkomponenten und gegebenenfalls zusätzlich mindestens einer Kupplerkomponente und gegebenenfalls zusätzlich mindestens einem Oxidationsmittel verstanden.

Die erfindungsgemäßen Entwicklerkomponenten werden bevorzugt aus der Gruppe ausgewählt, die gebildet wird aus p-Phenylendiaminderivaten, zweikernigen Entwicklerkomponenten, p-Aminophenol und seinen Derivaten, Pyrimidinderivaten, Pyrazolderivaten sowie Pyrazozlopyrimidinderivaten und den physiologisch verträglichen Salzen dieser Verbindungen.

Besonders bevorzugte erfindungsgemäße Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäß bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Bei den erfindungsgemäßen direktziehenden Farbstoffen handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Erfindungsgemäße anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Erfindungsgemäße kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Erfindungsgemäße nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin werden als direktziehende Farbstoffe auch in der Natur vorkommende Farbstoffe eingesetzt, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Im Rahmen dieser Ausführungsform kann das erfindungsgemäße Mittel zusätzlich mindestens eine weitere farbverändernde Komponente enthalten. Eine farbverändernde Komponente im Sinne der Erfindung ist eine Komponente, bei deren Anwendung die Farbe keratinhaltiger Fasern sichtbar verändert wird.

Die zusätzliche farbverändernde Komponente wird bevorzugt ausgewählt
(1) aus Oxofarbstoffvorprodukten
   und/oder
(2) aus mindestens einer Vorstufe naturanaloger Farbstoffe und/oder
(3) aus mindestens einem Oxidationsmittel und mindestens einem Bleichverstärker.

Als Oxofarbstoffvorprodukte kommt bevorzugt eine Kombination aus
- mindestens einer Verbindung, die mindestens eine reaktive Carbonylgruppe enthält (Komponente (Oxo 1)) mit mindestens einer Verbindung (Komponente Oxo2)
- Verbindungen, ausgewählt aus
   (Oxo2a) CH-aciden Verbindungen
      und/oder aus
   (Oxo2b) Verbindungen mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterozyklischen Verbindungen und aromatischen Hydroxyverbindungen
   zum Einsatz.
Reaktive Carbonylverbindungen als Komponente (Oxo1) besitzen im Sinne der Erfindung mindestens eine Carbonylgruppe als reaktive Gruppe, welche mit der Komponente (Oxo2) unter Ausbildung einer kovalenten Bindung reagiert. Bevorzugte reaktive Carbonylverbindungen sind ausgewählt aus Verbindungen die mindestens eine Formylgruppe und/oder mindestens eine Ketogruppe, insbesondere mindestens eine Formylgruppe, tragen. Ferner sind erfindungsgemäß auch solche Verbindungen als Komponente (Oxo1) verwendbar, in denen die reaktive Carbonylgruppe derart derivatisiert bzw. maskiert ist, dass die Reaktivität des Kohlenstoffatoms der derivatisierten Carbonylgruppe gegenüber der Komponente (Oxo2) stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente (Oxo1) leitet sich in diesem Fall c) von einem Aldehyd ab)
an das Kohlenstoffatom der Carbonylgruppe der reaktiven Carbonylverbindung.

Die voranstehend genannten Verbindungen der Komponente (Oxo1) sowie der Komponente (Oxo2) werden, wenn sie zum Einsatz kommen, jeweils vorzugsweise in einer Menge von 0,03 bis 65 mmol, insbesondere von 1 bis 40 mmol, bezogen auf 100 g des gesamten Mittels, verwendet.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

In einer weiteren bevorzguten Ausführungsform der vorliegenden Erfindung enthalten die Mittel als zusätzliche farbverändernde Verbindung mindestens ein Oxidationsmittel und mindestens einen Bleichverstärker. Diese Mittel werden üblicherweise Blondiermittel genannt.

Zum Blondieren menschlicher Haare - insbesondere für die Strähnchenapplikation -werden üblicherweise feste oder pastenförmige Zubereitungen mit Bleichverstärkern unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Bleichverstärker werden bevorzugt in Blondiermitteln zur Steigerung der Blondierwirkung des Oxidationsmittels eingesetzt. Die bevorzugt verwendbaren Oxidationsmittel finden später Erwähnung (vide infra).

Bleichverstärker sind bevorzugt Peroxoverbindungen. Unter die erfindungsgemäß bleichverstärkenden Peroxoverbindungen fallen keine Anlagerungsprodukte von Wasserstoffperoxyd an andere Komponenten und auch nicht Wasserstoffperoxyd selbst. Die Auswahl der Peroxoverbindungen unterliegt darüber hinaus keinen Beschränkungen. Bevorzugte Peroxoverbindungen sind Peroxidisulfatsalze, Persulfatsalze, (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Bleichverstärker sind in den erfindungsgemäßen, kosmetischen Mitteln bevorzugt in Mengen von 5 bis 30 Gew.-%, insbesondere in Mengen von 8 bis 20 Gew.-%, enthalten.

Als weitere wichtige Komponente enthalten Blondiermittel bevorzugt mindestens ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, -carbonate, - hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.

Blondiermittel enthalten Alkalisierungsmittel bevorzugt in Mengen von 5 bis 25 Gew.-%, insbesondere 10 bis 20 Gew.-%.

Es ist im Rahmen einer weiteren Ausführungsform bevorzugt dem Mittel, enthaltend die erfindungsgemäße Wirkstoffkombination zusätzlich mindestens ein Oxidationsmittel zuzusetzen, insbesondere dann, wenn die erfindungsgemäßen Mittel als oxidative Färbemittel konfektioniert werden. Werden die erfindungsgemäßen Mittel als Bleichmittel konfektioniert, enthalten sie zwangsweise mindestens ein entsprechendes Oxidationsmittel(vide supra).Die Oxidationsmittel im Sinne der Erfindung sind von Luftsauerstoff verschieden und besitzen ein solches Oxidationspotenzial, das es ermöglicht, das natürliche Farbpigment Melanin oxidativ aufzuhellen und/oder ein Oxidationsfarbstoffvorprodukt vom Entwicklertyp zu oxidieren.

Solche Oxidationsmittel sind beispielsweise Wasserstoffperoxid, Anlagerungsprodukte des Wasserstoffperoxids, organische Percarbonsäuren oder organische Peroxide. Als Oxidationsmittel kommt bevorzugt Wasserstoffperoxid und/oder mindestens ein Anlagerungsprodukt davon, insbesondere an anorganische oder organische Verbindungen, in Frage. Die geeigneten Anlagerungsprodukte von Wasserstoffperoxid werden wiederum bevorzugt ausgewählt aus mindestens einer Verbindung der Gruppe, bestehend aus Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid.

Erfindungsgemäß können die Oxidationsmittel auch zusammen mit einem Katalysator Anwendung finden. Der Katalysator aktiviert die Oxidation des Substrats, wie beispielsweise die Oxidation der Oxidationsfarbstoffvorprodukte oder des Melanins. Solche Katalysatoren sind z.B. Metallionen, lodide, Chinone oder bestimmte Enzyme.

Das Oxidationsmittel ist bevorzugt in einer Menge von 1,0 bis 15 Gew.-%, insbesondere von 2,0 bis 6,0 Gew.-%, jeweils bezogen auf das Gewicht des anwendungsbereiten Mittels, in einem erfindungsgemäß verwendbaren Mittel enthalten.

Ein oxidatives Färbemittel wird zweckmäßigerweise unmittelbar vor der Anwendung durch Mischung einer Zubereitung enthaltend mindestens ein Oxidationsmittel mit einer Zubereitung, enthaltend die Farbstoffvorprodukte, hergestellt. Das dabei entstehende gebrauchsfertige Haarfärbepräparat sollte bevorzugt einen pH-Wert im Bereich von 5 bis 14, insbesondere von 7 bis 12, aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde. Die erfindungsgemäße Wirkstoffkombination kann dabei in der oxidationemittelhaltigen Zubereitung oder in der Zubereitung, die die Oxidationsfarbstoffvorstufen enthält, konfektioniert sein. Letztere Art der Konfektionierung der Wirkstoffkombination in einem 2-Komponenten Färbemittel ist bevorzugt.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin hat es sich als vorteilhaft erwiesen, wenn die erfindungsgemäßen Mittel aller vorgenannten Ausführungsformen zusätzlich mindestens einen Pflegestoff enthalten, der sich von den Verbindungen der erfindungsgemäßen Wirkstoffkombination unterscheidet.

Die erfindungsgemäßen Mittel können neben den erfindungswesentlichen Komponenten weiterhin alle für solche Zubereitungen bekannten Wirk-, Zusatz- und Hilfsstoffe enthalten.

In vielen Fällen enthalten die Mittel bevorzugt mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Hinsichtlich der kationischen Tenside sei an dieser Stelle auf die obigen Ausführungen verwiesen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO(⁻)- oder -SO₃(⁻)-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine-COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Ferner können die erfindungsgemäßen Mittel weitere Wirk-, Hilfs- und Zusatzstoffe, wie beispielsweise enthalten
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine, Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol, Cholesterin,
- Konsistenzgeber wie Zuckerester, Polyolester oder Polyolalkylether,
- Fette und Wachse wie Walrat, Bienenwachs, Montanwachs und Paraffine, Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere Perlglanzmittel wie Ethylenglykolmono- und -distearat sowie PEG-3-distearat,
- Konservierungsmittel,
- Stabilisierungsmittel für Wassserstoffperoxid und andere Oxidationsmittel,
- Treibmittel wie Propan-Butan-Gemische, N₂O, Dimethylether, CO₂ und Luft
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der vorliegenden Erfindung ist ein Verfahren zur Behandlung keratinischer Fasern, bei dem eines der Mittel des ersten Erfindungsgegenstandes auf die Fasern aufgetragen wird und nach einer Einwirkzeit wieder abgespült wird.

Ein dritter Gegenstand der vorliegenden Erfindung ist die Verwendung einer Wirkstoffkombination aus
(A) mindestens eine Biotinverbindung der Formel (I) 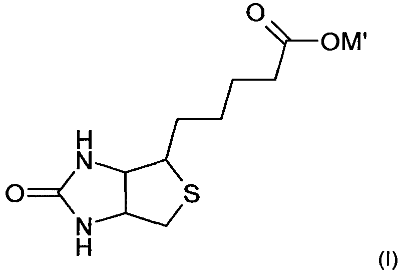 worin M' für ein ein Wasserstoffatom, eine (C₁ bis C₆)-Alkylgruppe, eine (C₂ bis C₆)-Hydroxyalkylgruppe, eine (C₂ bis C₆)-Alkenylgruppe, ein Äquivalent eines ein- oder mehrwertigen Kations steht und
(B) mindestens einer gegebenenfalls hydratisierten SiO₂-Verbindung in Färbemitteln für keratinische Fasern
   - zur Verbesserung der Farbintensität
   - zur Verbesserung der Farbechtheit.

### Beispiele

Die in den Beispielrezepturen genannten Mengenangaben verstehen sich, soweit nichts anderes vermerkt ist, als Gewichtsprozent.

### 33. oxidative Färbemittel

Die im Folgenden beschriebenen Färbecremes V bis VIII und XII bis XVI (Tabellen 33.2 und 33.4) wurden hergestellt und jeweils unmittelbar vor der Anwendung im Verhältnis 1:1 mit der folgenden Oxidationsmittelzubereitung vermischt:

| Rohstoff | Menge |
|---|---|
| Dipicolinsäure | 0,1 |
| 50%ige KOH | 0,3 |
| Natriumbenzoat | 0,04 |
| Natriumpyrophosphat | 0,1 |
| Turpinal® SL | 0,4 |
| 1,2-Propylenglykol | 0,4 |
| Cetyl/Stearylalkohol 50 : 50 | 4,0 |
| Dehyquart® B | 0,75 |
| Emulgin® B2 | 1,2 |
| Paraffinöl | 0,3 |
| 50%ige H₂O₂ | 12,2 |
| Wasser | ad 100,0 |

Die resultierende Anwendungsmischung wurde auf Strähnen aus Büffelbauchhaar aufgetragen und dort für 30 Minuten bei Raumtemperatur belassen. Anschließend wurden die Fasern gründlich mit Wasser gespült und die Färbungen ermittelt.

**Tabelle 33.2:**

| Rohstoff | Färbecreme V | Färbecreme VI | Färbecreme VII | Färbecreme VIII |
|---|---|---|---|---|
| Ammoniumcarbopollösung (1% in Wasser) | 18,0 | 18,0 | 18,0 | 18,0 |
| Ammonium Rohagit® (6% in Wasser) | 15,0 | 15,0 | 15,0 | 15,0 |
| Eumulgin® KE 2602 | 0,7 | 0,7 | 0,7 | 0,7 |
| Kaliumoleat | 4,4 | 4,4 | 4,4 | 4,4 |
| Kalium Castorate | 0,6 | 0,6 | 0,6 | 0,6 |
| Plantacare® 2000 UP | 3,0 | 3,0 | 3,0 | 3,0 |
| Titandioxid | 0,5 | 0,5 | 0,5 | 0,5 |
| Lanette® O | 12,0 | 12,0 | 12,0 | 12,0 |
| Cetiol® V | 3,0 | 3,0 | 3,0 | 3,0 |
| Cutina® GMS V | 2,0 | 2,0 | 2,0 | 2,0 |
| Kalilauge 50% | 0,9 | 0,9 | 0,9 | 0,9 |
| EDTA, Tetranatriumsalz | 0,2 | 0,2 | 0,2 | 0,2 |
| Natriumsulfit | 0,2 | 0,2 | 0,2 | 0,2 |
| Ascorbinsäure | 0,05 | 0,05 | 0,05 | 0,05 |
| Mirapol® A 15 | 1,5 | 1,5 | 1,5 | 1,5 |
| Parfum | 0,3 | 0,3 | 0,3 | 0,3 |
| Phospholipid® EFA | 0,1 | 0,1 | 0,1 | 0,1 |
| Puricare® LS 9658 | -- | 1,0 | -- | 1,0 |
| Kieselsäure | 0,25 | 0,25 | 0,25 | 0,25 |
| Biotin | 0,005 | 0,005 | 0,005 | 0,005 |
| L-Carnitin-L-tartrat | -- | -- | 0,2 | 0,2 |
| p-Aminophenol | 0,02 | 0,2 | -- | -- |
| p-Phenylendiamin | 1,15 | -- | -- | -- |
| 1-(2-Hydroxyethyl)-4,5-diaminopyrazol | -- | -- | 1,5 | 0,93 |
| p-Toluylendiamin | -- | 0,97 | 0,18 | -- |
| HC Blue No. 7 | -- | -- | -- | 0,40 |
| Resorcin | 0,60 | 0,45 | -- | -- |
| o-Aminophenol | 0,06 | -- | -- | -- |
| m-Aminophenol | 0,15 | 0,09 | 0,04 | --- |
| 2,6 Diaminopyridin | 0,011 | -- | -- | -- |
| 5-Amino-2-methylphenol | -- | 0,06 | 0,80 | -- |
| 1-Naphthol | -- | -- | -- | 0,40 |
| 2-Amino-4-hydroxyethylaminoanisol sulfat | -- | -- | -- | 0,13 |
| 2-Nitro-p-phenylendiamin | -- | 0,20 | -- | -- |
| Ammoniaklösung (25% in Wasser) | ad 10,2 | ad 10,4 | ad 10,3 | ad 10,5 |
| Wasser | ad 100,0 | ad 100,0 | ad 100,0 | ad 100,0 |
| Nuance | Hellbraun | Kupfer | Rot | Violett |

**Tabelle 33.4:**

| Rohstoff | Färbecreme XIII | Färbecreme XIV | Färbecreme XV | Färbecreme XVI |
|---|---|---|---|---|
| Ammoniumcarbopollösung (1% in Wasser) | 15,0 | 15,0 | 15,0 | 15,0 |
| Lanette® E | 0,7 | 0,7 | 0,7 | 0,7 |
| Laurylethersulfat (27% in Wasser) | 4,4 | 4,4 | 4,4 | 4,4 |
| PEG 600 | 0,6 | 0,6 | 0,6 | 0,6 |
| Kaliumoleat (12.5% in Wasser) | 3,0 | 3,0 | 3,0 | 3,0 |
| Titandioxid | 0,15 | 0,15 | 0,15 | 0,15 |
| Cetylstearylalcohol 50/50 | 12,0 | 12,0 | 12,0 | 12,0 |
| Eumulgin® B2 | 3,0 | 3,0 | 3,0 | 3,0 |
| Eutanol® G | 2,0 | 2,0 | 2,0 | 2,0 |
| Cutina® AGS | 2,0 | 2,0 | 2,0 | 2,0 |
| Cutina® GMS-SE | 2,0 | 2,0 | 2,0 | 2,0 |
| XF42-B1989® | 1,5 | 1,5 | 1,5 | 1,5 |
| Glycyrrhiza Extracted Powder | 0,2 | 0,2 | 0,2 | 0,2 |
| Potassium Carbonate, Anhydrous | 1,0 | 1,0 | 1,0 | 1,0 |
| Diammoniumhydrogenphosphate | 0,6 | 0,6 | 0,6 | 0,6 |
| EDTA, Tetranatriumsalz | 0,4 | 0,4 | 0,4 | 0,4 |
| Natriumsulfit | 0,1 | 0,1 | 0,1 | 0,1 |
| Ascorbinsäure | 0,1 | 0,1 | 0,1 | 0,1 |
| Merquat Plus® 3330 | 1,5 | 1,5 | 1,5 | 1,5 |
| Parfum | 0,5 | 0,5 | 0,5 | 0,5 |
| Puricare® LS 9658 | -- | 0,3 | -- | 0,3 |
| Kieselsäure | 0,2 | 0,2 | 0,2 | 0,2 |
| Biotin | 0,001 | 0,001 | 0,001 | 0,001 |
| L-Carnitin-L-tartrat | -- | -- | 2,0 | 2,0 |
| p-Aminophenol | 0,02 | 0,20 | -- | -- |
| p-Phenylendiamin | 1,15 | -- | -- | -- |
| 1-(2-Hydroxyethyl)-4,5-diaminopyrazol | -- | -- | 1,50 | 0,93 |
| p-Toluylendiamin | -- | 0,97 | 0,18 | -- |
| HC Blue No. 7 | -- | -- | -- | 0,40 |
| Resorcin | 0,60 | 0,45 | -- | -- |
| o-Aminophenol | 0,06 | -- | -- | -- |
| m-Aminophenol | 0,15 | 0,09 | 0,04 | -- |
| 2,6 Diaminopyridin | 0,01 | -- | -- | -- |
| 5-Amino-2-methylphenol | -- | 0,06 | 0,80 | -- |
| 1-Naphthol | -- | -- | -- | 0,40 |
| 2-Amino-4-hydroxyethylaminoanisol Sulfat | -- | - | -- | 0,13 |
| 2-Nitro-p-phenylendiamin | -- | 0,20 | -- | -- |
| Kalilauge (50% in Wasser) | ad pH 9,1 | ad pH 9,1 | ad pH 9,1 | ad pH 9,1 |
| Wasser | ad 100,0 | ad 100,0 | ad 100,0 | ad 100,0 |
| Nuance | Hellbraun | Kupfer | Rot | Violett |

- Ammoniumcarbopollsg.: Lösung eines Ammoniumsalzes eines Methacrylsäure-Methylacrylat-Copolymeren (INCI-Bezeichnung: Ammonium Polyacrylate) (Röhm GmbH)
- Ammoniumrohagitlösung: Lösung eines Ammoniumsalzes eines Acrylsäure-Polymeren (INCI-Bezeichnung: Ammonium Acrylates Copolymer) (Goodrich)
- Cetiol® V: Ölsäuredecylester (INCI-Bezeichnung: Decyl Oleate) (Cognis)
- Cutina® AGS: Ethylenglykoldistearat (INCI-Bezeichnung: Glycol Distearate) (Cognis)
- Cutina® GMS-SE: INCI-Bezeichnung: Glyceryl Stearate SE (Cognis)
- Cutina® GMS-V: Glycerinmono/dipalmitat/stearat (INCI-Bezeichnung: Glyceryl Stearate) (Cognis)
- Dehyquart® B: Stearyltrimethylammoniumchlorid (ca. 60-66% Aktivsubstanzgehalt; INCI-Bezeichnung: Steartrimonium Chloride) (Cognis)
- Eumulgin® B2: Cetylstearylalkohol mit ca. 20 EO-Einheiten (INCI-Bezeichnung: Ceteareth-20) (Cognis)
- Eumulgin® KE 2602: ethoxylierter Oleylalkohol (INCI-Bezeichnung: Oleth-7) (Cognis)
- Eutanol® G: 2-Octyldodecylalkohol (INCI-Bezeichnung: Octyldodecanol) (Cognis)
- Glycyrrhiza Extr. Powder: Licorice-Extrakt (INCI-Bezeichnung: Glycyrrhiza Glabra (Licorice) Root Extract) (Maruzen)
- HC Blue No.7: INCI-Bezeichnung: 6-Methoxy-2-methylamino-3-aminopyridine HCl
- Lanette® E: Fettalkoholsulfat-Natrium-Salz (ca. 90-96% Aktivsubstanz-gehalt; INCI-Bezeichnung: Sodium Cetearyl Sulfate) (Cognis)
- Lanette® O: C₁₆₋₁₈-Fettalkohol (INCI-Bezeichnung: Cetearyl Alcohol) (Cognis)
- Merquat® Plus 3330: Dimethyldiallylammoniumchlorid Acrylsäure Acrylamid Terpolymer (ca. 9,5% Festkörper in Wasser; INCI-Bezeichnung: Polyquaternium-39) (Ondeo-Nalco)
- Mirapol® A 15: Poly[N-(3-(dimethylammonium)propyl]-N'-[3-ethylenoxyethylendimethyl-ammonium)-propyl]-harnstoff-di-chlorid (ca. 64% Festkörper in Wasser; INCI-Bezeichnung: Polyquaternium-2) (Rhodia)
- Phopholipid® EFA: (ca. 30% Festkörper in Wasser/Propylenglykol; INCI-Bezeichnung:Linoleamidopropyl PG-Dimonium Chloride Phosphate) (Uniqema)
- Plantacare® 2000 UP: C₈₋₁₆ Alkylglucosid (ca. 51-55% Aktivsubstanzgehalt in Wasser; INCI-Bezeichnung: Decyl Glucoside, Aqua (Water)) (Cognis)
- Puricare® LS 9658: Moringa Pterygosperma Extrakt, in Wasser/Glycerin (ca. 1%ige Lösung; INCI-Bezeichnung: Water, Glycerine, Moringa Pterygosperma Seed Extract) (Cognis)
- Turpinal® SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- XF42-B1989®: aminofunktionelles Silikon (INCI-Bezeichnung: Amodimethicone) (GE Bayer Silicones)

## Patentansprüche

1. Mittel zur Behandlung keratinischer Fasern, insbesondere menschlicher Haare, das in einem kosmetisch akzeptablen Träger
(A) mindestens eine Biotinverbindung der Formel (I) 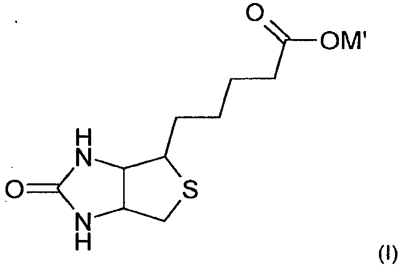 worin M' für ein Wasserstoffatom, eine (C₁ bis C₆)-Alkylgruppe, eine (C₂ bis C₆)-Hydroxyalkylgruppe, eine (C₂ bis C₆)-Alkenylgruppe, ein Äquivalent eines ein- oder mehrwertigen Kations steht,
wobei die Biotinverbindung in einer Menge von 0,0001 Gew.-% bis 0,01 Gew.-%, bezogen auf das Gewicht des Mittels, enthalten ist
und
(B) mindestens eine gegebenenfalls hydratisierte SiO₂-Verbindung enthält,
**dadurch gekennzeichnet, dass** zusätzlich mindestens ein Oxidationsfarbstoffvorprodukt und/oder mindestens ein direktziehender Farbstoff enthalten ist.

2. Mittel nach Anspruch 1, **dadurch gekennzeichnet, dass** die Biotinverbindungen in einer Menge von 0,0005 Gew.-% bis 0,01 Gew.-%, bezogen auf das Gewicht des Mittels, enthalten sind.

3. Mittel nach einem der Ansprüche 1 oder 2, **dadurch gekennzeichnet, dass** die gegebenenfalls hydratisierte SiO₂-Verbindungen ausgewählt werden aus
(i) polymeren Kieselsäuren und/oder
(ii) Wasserglas.

4. Mittel nach einem der Ansprüche 1 bis 3, **dadurch gekennzeichnet, dass** die gegebenenfalls hydratisierten SiO₂-Verbindungen in einer Menge von 0,005 Gew.-% bis 5 Gew.-%, bevorzugt in Mengen von 0,05 Gew.-% bis 2 Gew.-%, besonders bevorzugt in Mengen von 0,1 Gew.-% bis 1 Gew.-%, und ganz besonders bevorzugt in Mengen von 0,2 Gew.-% bis 1,0 Gew.-%, jeweils bezogen auf das Gewicht des Mittels, enthalten sind.

5. Mittel nach einem der Ansprüche 1 bis 4, **dadurch gekennzeichnet, dass** mindestens ein Protein des Samens der Gattung der Moringagewächse, insbesondere aus dem Samen der Moringa oleifera, enthalten ist.

6. Mittel nach einem der Ansprüche 1 bis 5, **dadurch gekennzeichnet, dass** es zusätzlich mindestens eine Verbindung der Formel (II) enthält 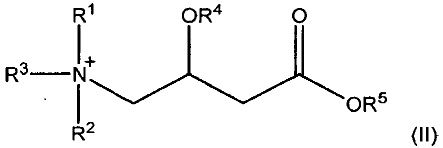 worin
R¹, R² und R³ unabhängig voneinander für eine C₁- bis C₄-Alkylgruppe oder eine C₂- bis C₄-Hydroxyalkylgruppe stehen,
R⁴ ein Wasserstoffatom, eine C₁- bis C₄-Alkylgruppe oder eine C₂- bis C₆-Acylgruppe bedeutet,
R⁵ für ein Wasserstoffatom, eine negative Ladung oder eine C₁- bis C₄-Alkylgruppe steht.

7. Mittel nach Anspruch 6, **dadurch gekennzeichnet, dass** die Verbindung gemäß Formel (II) mindestens eine Verbindung ist, ausgewählt aus der Gruppe L-Carnitin, D-Carnitin, D,L-Carnitin, L-Carnitintartrat, D-Carnitintartrat, D,L-Carnitintartrat, L-Carnitinlactat, D-Carnitinlactat, D,L-Carnitinlactat, L-Carnitincitrat, D-Carnitincitrat, D,L-Carnitincitrat, L-Acetylcarnitin, D-Acetylcarnitin und D,L-Acetylcarnitin.

8. Mittel nach einem der Ansprüche 6 oder 7, **dadurch gekennzeichnet, dass** die Verbindungen der Formel (II) in einer Menge von 0,001 bis 10 Gew.-%, besonders bevorzugt von 0,01 bis 5 Gew.-%, ganz besonders bevorzugt von 0,1 bis 2 Gew.-%, jeweils bezogen auf das Gewicht des Mittels, enthalten sind.

9. Mittel nach einem der Ansprüche 1 bis 8, **dadurch gekennzeichnet, dass** es zusätzlich mindestens eine keratinreduzierende Verbindung enthält.

10. Mittel nach einem der Ansprüche 1 bis 8, **dadurch gekennzeichnet, dass** zusätzlich mindestens ein Oxidationsmittel, insbesondere Wasserstoffperoxid, enthalten ist.

11. Verfahren zur Behandlung keratinischer Fasern, insbesondere menschlicher Haare, **dadurch gekennzeichnet, dass** ein Mittel nach einem der Ansprüche 1 bis 10 auf die Fasern aufgetragen wird und nach einer Einwirkzeit wieder abgespült wird.

12. Verwendung einer Wirkstoffkombination aus
(A) mindestens einer Biotinverbindung der Formel (I) 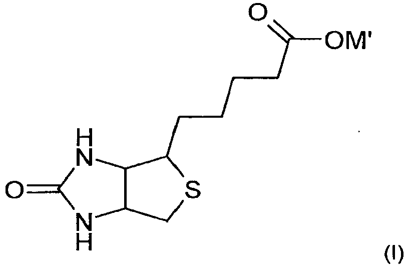 worin M' für ein Wasserstoffatom, eine (C₁ bis C₆)-Alkylgruppe, eine (C₂ bis C₆)-Hydroxyalkylgruppe; eine (C₂ bis C₆)-Alkenylgruppe, ein Äquivalent eines ein- oder mehrwertigen Kations steht
und
(B) mindestens einer gegebenenfalls hydratisierten SiO₂-Verbindung
in Färbemitteln für keratinische Fasern
- zur Verbesserung der Farbintensität
- zur Verbesserung der Farbechtheit.

## Claims

1. An agent for treating keratin fibers, in particular human hair, containing, in a cosmetically acceptable carrier,
(A) at least one biotin compound of formula (I) 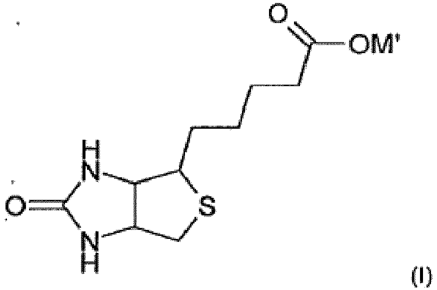 in which M' represents a hydrogen atom, a (C₁ to C₆) alkyl group, a (C₂ to C₆) hydroxyalkyl group, a (C₂ to C₆) alkenyl group, an equivalent of a mono- or polyvalent cation,
the biotin compound being contained in an amount of from 0.0001 wt.% to 0.01 wt.%, based on the weight of the agent,
and
(B) at least one optionally hydrated SiO₂ compound, **characterized in that** at least one oxidation dye precursor and/or at least one direct dye is additionally contained.

2. The agent according to claim 1, **characterized in that** the biotin compounds are contained in an amount of from 0.0005 wt.% to 0.01 wt.%, based on the weight of the agent.

3. The agent according to one of claims 1 or 2, **characterized in that** the optionally hydrated SiO₂ compounds are selected from
(i) polymeric silicic acids and/or
(ii) water glass.

4. The agent according to one of claims 1 to 3, **characterized in that** the optionally hydrated SiO₂ compounds are contained in an amount of from 0.005 wt.% to 5 wt.%, preferably in amounts of from 0.05 wt.% to 2 wt.%, particularly preferably in amounts of from 0.1 wt.% to 1 wt.%, and most particularly preferably in amounts of from 0.2 wt.% to 1.0 wt.%, in each case based on the weight of the agent.

5. The agent according to one of claims 1 to 4, **characterized in that** at least one protein of the seed of the moringa plant genus, in particular from the Moringa oleifera seed, is contained.

6. The agent according to one of claims 1 to 5, **characterized in that** it additionally contains at least one compound of formula (II) 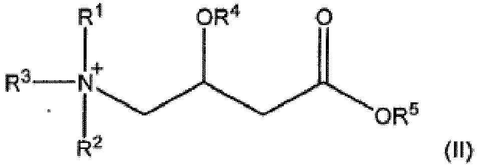 in which
R¹, R² and R³ represent, independently of one another, a C₁ to C₄ alkyl group or a C₂ to C₄ hydroxyalkyl group,
R⁴ signifies a hydrogen atom, a C₁ to C₄ alkyl group or a C₂ to C₆ acyl group,
R⁵ represents a hydrogen atom, a negative charge or a C₁ to C₄ alkyl group.

7. The agent according to claim 6, **characterized in that** the compound according to formula (II) is at least one compound, selected from the group of L-carnitine, D-carnitine, D,L-carnitine, L-carnitine tartrate, D-carnitine tartrate, D,L-carnitine tartrate, L-carnitine lactate, D-carnitine lactate, D,L-carnitine lactate, L-carnitine citrate, D-carnitine citrate, D,L-carnitine citrate, L-acetyl carnitine, D-acetyl carnitine and D,L-acetyl carnitine.

8. The agent according to one of claims 6 or 7, **characterized in that** the compounds of formula (II) are contained in an amount of from 0.001 to 10 wt.%, particularly preferably from 0.01 to 5 wt.%, more particularly preferably from 0.1 to 2 wt.%, in each case based on the weight of the agent.

9. The agent according to one of claims 1 to 8, **characterized in that** it additionally contains at least one keratin-reducing compound.

10. The agent according to one of claims 1 to 8, **characterized in that** at least one oxidizing agent, in particular hydrogen peroxide, is additionally contained.

11. A method for treating keratin fibers, in particular human hair, **characterized in that** an agent according to one of claims 1 to 10 is applied to the fibers and rinsed off again after a reaction time.

12. The use of an active ingredient combination of
(A) at least one biotin compound of formula (I) 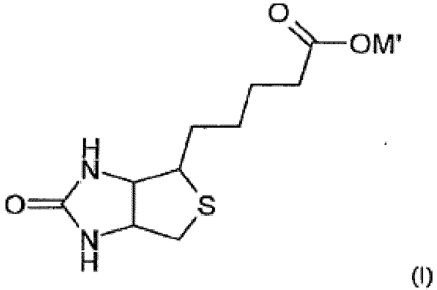 in which M' represents a hydrogen atom, a (C₁ to C₆) alkyl group, a (C₂ to C₆) hydroxyalkyl group, (C₂ to C₆) alkenyl group, an equivalent of a mono- or polyvalent cation
and
(B) at least one optionally hydrated SiO₂ compound
in coloring agents for keratin fibers
- for improving the color intensity
- for improving the color fastness.

## Revendications

1. Agent de traitement de fibres kératiniques, en particulier de cheveux humains, qui contient, dans un support cosmétiquement acceptable
(A) au moins un composé de la biotine de formule (I) 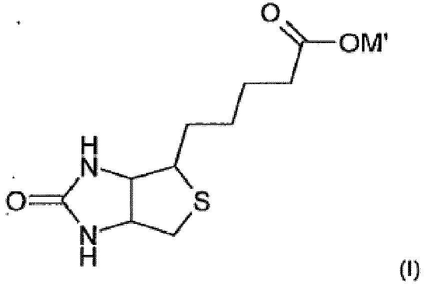 dans laquelle M' représente un atome d'hydrogène, un groupe alkyle C₁ à C₆, un groupe hydroxyalkyle C₂ à C₆, un groupe alcényle C₂ à C₆, un équivalent d'un cation monovalent ou polyvalent,
le composé de la biotine étant présent dans une quantité allant de 0,0001 % à 0,01 % en fonction de la masse de l'agent,
et
(B) au moins un composé du SiO₂ éventuellement hydraté, **caractérisé en ce qu'**au moins un précurseur de colorant d'oxydation et/ou au moins un colorant direct est en outre présent.

2. Agent selon la revendication 1, **caractérisé en ce que** les composés de la biotine sont présents dans une quantité allant de 0,0005 % à 0,01 % en fonction de la masse de l'agent.

3. Agent selon l'une des revendications 1 ou 2, **caractérisé en ce que** les composés du SiO₂ éventuellement hydratés sont choisis parmi
(i) les silices polymères et/ou
(ii) le silicate de sodium.

4. Agent selon l'une des revendications 1 à 3, **caractérisé en ce que** les composés du SiO₂ éventuellement hydratés sont présents dans une quantité allant de 0,005 % à 5 % en fonction de la masse, de préférence dans des quantités allant de 0,05 % à 2 % en fonction de la masse, de manière particulièrement préférée dans des quantités allant de 0,1 % en à 1 % en fonction de la masse, et de manière tout particulièrement préférée dans des quantités allant de 0,2 % à 1,0 % toujours en fonction de la masse de l'agent.

5. Agent selon l'une des revendications 1 à 4, **caractérisé en ce qu'**il contient au moins une protéine de la graine du genre Moringa, en particulier de la graine de Moringa oleifera.

6. Agent selon l'une des revendications 1 à 5, **caractérisé en ce qu'**il contient en outre au moins un composé de la formule (II) 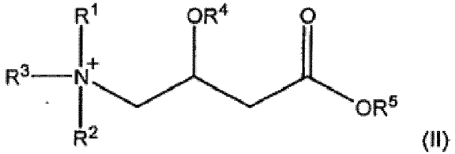 dans laquelle
R¹, R² et R³ représentent indépendamment chacun un groupe alkyle C₁ à C₄ ou un groupe hydroxyalkyle C₂ à C₄,
R⁴ représente un atome d'hydrogène, un groupe alkyle C₁ à C₄ ou un groupe acyle C₂ à C₆,
R⁵ représente un atome d'hydrogène, une charge négative ou un groupe alkyle C₁ à C₄.

7. Agent selon la revendication 6, **caractérisé en ce que** le composé selon la formule (II) est au moins un composé choisi dans le groupe comprenant la L-carnitine, la D-carnitine, la D,L-carnitine, le tartrate L-carnitine, le tartrate de D-carnitine, le tartrate D,L-carnitine, le lactate L-carnitine, le lactate D-carnitine, le lactate D,L- carnitine, le citrate L-carnitine, le citrate D-carnitine, le citrate D,L-carnitine, la L-acétylcarnitine, la D-acétylcarnitine et la D, L-acétylcarnitine.

8. Agent selon l'une des revendications 6 ou 7, **caractérisé en ce que** les composés de la formule (II) sont présents dans une quantité allant de 0,001 à 10 % en fonction de la masse, de manière particulièrement préférée de 0,01 à 5 % en fonction de la masse, de manière tout particulièrement préférée de 0,1 à 2 % toujours en fonction de la masse de l'agent.

9. Agent selon l'une des revendications 1 à 8, **caractérisé en ce qu'**il contient en outre au moins un composé réducteur de la kératine.

10. Agent selon l'une des revendications 1 à 8, **caractérisé en ce qu'**au moins un agent oxydant, en particulier du peroxyde d'hydrogène, est en outre présent

11. Procédé de traitement de fibres kératiniques, en particulier de cheveux humains, **caractérisé en ce qu'**un agent selon l'une des revendications 1 à 10 est appliqué sur les fibres et est rincé à nouveau après un temps d'action.

12. Utilisation d'une combinaison d'ingrédients actifs comprenant
(A) au moins un composé de la biotine de la formule (I) 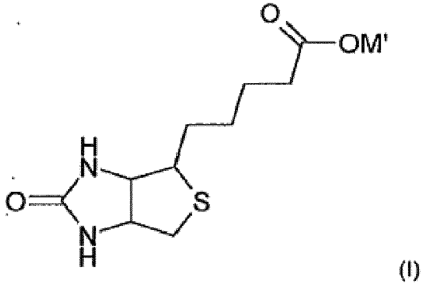 dans laquelle M' représente un atome d'hydrogène, un groupe alkyle C₁ à C₆, un groupe hydroxyalkyle C₂ à C₆, un groupe alcényle C₂ à C₆, un équivalent d'un cation monovalent ou polyvalent,
et
(B) au moins un composé du SiO₂ éventuellement hydraté,
dans des agents de coloration de fibres kératinique
- pour améliorer l'intensité de la couleur
- pour améliorer la solidité de la couleur.
